# EUROPEAN PATENT SPECIFICATION

(11) **EP 3 787 532 B1**
(45) Date of publication and mention of the grant of the patent: **13.12.2023**
(21) Application number: 19796100.6
(22) Date of filing: 15.04.2019
(51) Int. Cl.: A61B 17/34

(54) **PERICARDIAL SPACE ACCESS DEVICE**
VORRICHTUNG FÜR ZUGANG ZUM PERIKARDIALEN RAUM
DISPOSITIF D'ACCÈS À UN ESPACE PÉRICARDIQUE

(30) Priority: 30.04.2018 US 201862664785 P; 28.03.2019 US 201916368644
(43) Date of publication of application: 10.03.2021
(73) Proprietor: Khairkhahan, Sebastian, Palo Alto CA 94301 (US); Khairkhahan, Alexander, Palo Alto, CA 94301 (US)
(72) Inventor: Khairkhahan, Sebastian, Palo Alto CA 94301 (US); Khairkhahan, Alexander, Palo Alto, CA 94301 (US)
(74) Representative: Clark, Jane Anne
(86) International application number: PCT/US2019/027524
(87) International publication number: WO 2019/212733

(56) References cited:
- US-A1- 2003 093 104
- US-A1- 2007 135 883
- US-A1- 2008 294 174
- US-A1- 2010 105 981
- US-A1- 2012 095 434
- US-A1- 2012 253 386
- US-A1- 2016 213 462
- US-A1- 2017 143 322
- US-B2- 7 736 347
- US-B2- 8 075 532

## Description

### BACKGROUND OF THE INVENTION

1. Field of the Invention. The present invention relates generally to medical devices. More specifically, the present invention relates to devices for establishing access to a pericardial space.

The human heart is enveloped within a tissue structure referred to as the pericardium. The pericardium includes two major layers. The layer which lies immediately over the epicardial surface of the heart is referred to as the visceral pericardium. The second layer is formed as a sac around the visceral pericardium and is referred to as the parietal pericardium. Normally, the visceral and parietal pericardia lie in close contact with each other and are separated only by a thin layer of pericardial fluid. The space between the visceral and parietal pericardia is a "potential" space referred to as the pericardial space.

Access to the pericardial space is useful for a variety of purposes including the placement of epicardial pacing leads, drug delivery, transmyocardial access to the heart chambers, and of particular interest to the present invention, cardiac ablation. Surgical access can be obtained via an open sternotomy where the patient's sternum is divided and the parietal pericardium exposed. Such an approach, however, is highly traumatic, requiring general anesthesia and useful only under compelling circumstances.

Access to the pericardial space can also be achieved less invasively using a thoracoscopic approach where access is established via an introducer sheath positioned through the parietal pericardium and into the pericardial space over the visceral pericardium.

It would be desirable to provide additional and improved methods and apparatus for the minimally invasive access to a patient's pericardial space. The methods and devices should be suitable for a wide variety of minimally invasive approaches to the pericardium, including at least intercostal/transthoracic and subxiphoid approaches, and the like. The methods and devices should further provide for secure and stable capture of the parietal pericardium and permit the opening of a large space or volume between the parietal and visceral pericardia. At least some of these objectives will be met by the inventions described and claimed below.

2. Listing of the Background Art. Relevant US Patents and Patent Publications include: US2008/294174; US9259317; US9179932; US8986278; US8603031; US8460181; US8317810; US7881810; US7736347; US6835193; US6156009; US2017/119435; US2015/230699; and US2005/234507.

US 2007/0143322 describes a tissue engagement device that includes a sheath, a first arm, a second arm and an actuation cannula. At least a proximal portion of each arm is within the sheath. The actuation cannula is also within the sheath and can move within the sheath between a retracted position and an extended position. The actuation cannula moves at least a portion of each of the first and second arms into a region between an exterior surface of the actuation cannula and an interior surface of the sheath as the actuation cannula moves to the extended position.

### SUMMARY OF THE INVENTION

The present invention is set out in the appended claims. Disclosed herein are devices, systems, and methods for initiating and establishing pericardial access for performing intra-pericardial procedures, trans-myocardial procedures including fluid withdrawal, drug delivery, diagnostic and therapeutic electrophysiology procedures, pacemaker lead implantation, defibrillator lead placement, placement of the left ventricular assist devices, placement of the arterial bypass grafts, in situ bypass, i.e., coronary artery-venous fistulae, placement of drug delivery depots, closure of the left arterial appendage, and the and the like. The present invention is particularly useful for cardiac ablation procedures when ablation catheters can be advanced over guidewires placed into the pericardial space using the devices of the present invention. The surgical methods disclosed do not form part of the claimed invention.

In a first aspect, the present disclosure provides a pericardial access device which includes an outer tube, an inner tube, and an access needle. The outer tube has an axial passage which is configured to slidably receive the inner tube to allow a distal end of the inner tube to be axially extended and retracted relative to a distal end of the outer tube. A pericardial interface structure is formed or disposed on a distal end of the inner tube and typically includes a plurality of tissue-engaging tines, where the tines are configured to deploy from a constrained configuration to an expanded configuration as the pericardial interface structure is advanced distally from the distal end of the outer tube.

Typically, individual tissue-engaging tines are formed from an elastic or superelastic material and are in their "deployed" configuration, typically curved over an arc of 150° to 210°, when unconstrained. Prior to deployment, however, the tines are usually constrained within a distal region of the outer tube in a low profile configuration which facilitates introduction and removal of the pericardial access device from a target location, often including advancement through a laparoscopic, thoracoscopic, or similar minimally invasive introducing port. The access needle typically includes a tissue-penetrating tip on its distal end, where the access needle is slidably received in an axial passage of the inner tube. In this way, after the pericardial access device is introduced to a surgical target site, the inner tube can be advanced distally from or retracted proximally back into the outer tube, depending on the particular treatment protocol. The access needle can be advanced from the inner tube to penetrate a pericardial or other tissue site after the tissue-engaging tines are deployed in the desired manner.

In specific examples, the individual tissue-engaging tines are further configured to penetrate tissue as they are advanced from the distal end of the outer tube. In such instances, as described more fully below, tines may be used to engage and retract a parietal pericardium of the patient's heart in order to enlarge a pericardial space to perform procedures within the pericardial space. In alternative instances, however, the plurality of tissue-engaging tines may be configured to atraumatically engage tissue as they are advanced from a distal end of the outer tube. In such instances, the devices of the present disclosure will typically not be used to retract the parietal pericardium in order to enlarge a pericardial space prior to needle deployment into the pericardial space. In still other instances, the tissue-engaging tines may be configured to permit both tissue-penetrating engagement with the parietal pericardium and atraumatic engagement with the parietal pericardium so that the same access device can be used in different pericardial space access protocols.

In specific embodiments of the devices of the present disclosure, the plurality of tissue-engaging tines will be disposed about a periphery of a distal end of the inner tube. In such instances, each tine will typically be straightened when constrained within the outer tube and will evert radially outwardly as the tine is advanced from the distal end of the outer tube. In some instances, the tines will have tissue-penetrating tips so that they will be engaged against the parietal pericardium to penetrate and capture the parietal pericardium in order to retract it to enlarge the pericardial space. In other instances, the leading or distal surfaces of the everted tines may provide an atraumatic tip for engaging the parietal pericardium without tissue penetration. In some instances, whether or not the tines penetrate the parietal pericardium will depend on whether or not the tips of the tines are engaged against the parietal pericardium while they are being advanced from the other tube.

In preferred embodiments of the present disclosure, the outer tubes may comprise a cannula having a blunt end such that the cannula can be engaged directly against the parietal pericardium or other tissue surface as the inner tube is being advanced to deploy the tissue-engaging tines. The use of cannulas having a blunt tip to engage the parietal pericardium is particularly advantageous when the tines are being used to penetrate and capture the parietal pericardium in order to enlarge the pericardial space.

In other instances, the outer tube may possess a tissue-penetrating distal end. Such embodiments are useful for allowing the access system to self-penetrate the patient's skin in order to provide direct percutaneous access, e.g. sub-xiphoid or intercostal access to the patient's heart. In such instances, the outer tube will typically be retracted over the inner tube and atraumatic-tissue engaging tines or other structures will be deployed from the inner tube prior to engaging a pericardial surface and percutaneously advancing the tissue-penetrating tip of the outer tube through target tissue.

In still other embodiments and instances, the inner tube may have an adjustable needle-stop structure at its proximal end in order to control a penetration distance afforded the access needle tip. For example, a threaded end cap may be placed at a proximal end of the inner tube in order to provide an axially adjustable needle stop.

In a second aspect, the present disclosure provides a method for accessing and enlarging a pericardial space between a visceral pericardium and a parietal pericardium surrounding a patient's heart. The method comprises advancing an atraumatic end of a cannula against a target site on an exterior of the parietal pericardium. An inner tube is advanced through an axial passageway on the cannula to advance a plurality of tines from a distal end of the cannula, where individual tines evert and penetrate the parietal pericardium, resulting in capture of the parietal pericardium by the inner tube. After the parietal pericardium has been captured, the cannula and inner tube may be drawn proximally, i.e. in a direction away from the surface of the heart, to separate the parietal pericardium from the underlying visceral pericardium, resulting in enlargement of the pericardial space. After the pericardial space has been enlarged, an access needle may be advanced through an axial passage of the inner tube so that a distal tip of the access needle enters the enlarged pericardial space with minimal or no penetration of the visceral pericardium or underlying myocardium. Once in place, the needle has a lumen to provide an access path into the enlarged pericardial space to allow a variety of procedures, such as drug delivery, cardiac ablations, and other procedures as noted in the background above.

In particular aspects of the methods herein, a guidewire will typically be advanced through a lumen of the access needle to a target location in the parietal space. After the guidewire is in place, the pericardial access system, including the cannula, inner tube, and access needle, may be retracted or withdrawn over a proximal section of the guidewire, allowing an introducer sheet to be advanced over the guidewire and through the parietal pericardium to establish working access into the pericardial space.

In a third aspect, the present disclosure provides a second method for accessing a pericardial space between a visceral pericardium and a parietal pericardium. This second method comprises percutaneously advancing a tissue-penetrating end of a cannula (outer tube) into a patient's thorax. A tissue-penetrating distal tip of the cannula is then retracted proximally over an inner tube to cause or allow a plurality of tines to evert radially outwardly from a distal end of the inner tube to form an atraumatic interface over a target site on an exterior of the parietal pericardium. The inner tube is then advanced through the retracted cannula to engage the atraumatic interface against the target site on the exterior of the parietal pericardium. An access needle may then be advanced through an axial passage of the inner tube so that a distal tip of the needle passes through the parietal pericardium and enters the pericardial space with limited or no penetration of the visceral pericardium. The needle has an access lumen, preferably on a side of a distal tip of the needle immediately proximal to a tissue-penetration tip of the needle, to provide an access path into the pericardial space.

The needle may then be used to introduce a guidewire, introduce drugs, or for any other purpose conventionally performed within the pericardial space.

### BRIEF DESCRIPTION OF THE DRAWINGS

Fig. 1 is an exploded view of a pericardial access system.
Figs. 2A through 2D illustrate assembly and use of the components of the pericardial access system of Fig. 1.
Figs. 3A through 3E illustrate use of the pericardial access system of Fig. 1 for penetrating and enlarging a pericardial space in the heart of a patient.
Figs. 4A through 4E illustrate the advancement of a guidewire into a pericardial space of a patient enlarged by the methods and tools described herein for use in a variety of cardiac procedures.
Figs. 5A through 5D illustrate use of a guidewire which has been introduced by the methods disclosed for advancing a secondary guidewire around the pulmonary veins of a patient for use in cardiac ablation or other procedures.
Fig. 6 illustrates an alternative embodiment of a pericardial access system having a tissue-penetrating outer tube and an atraumatic pericardial interface structure.
Figs. 7A through 7D illustrate use of the pericardial access system of Fig. 6 for penetrating a pericardial space without enlargement and advancing a guidewire into that pericardial space.

### DETAILED DESCRIPTION

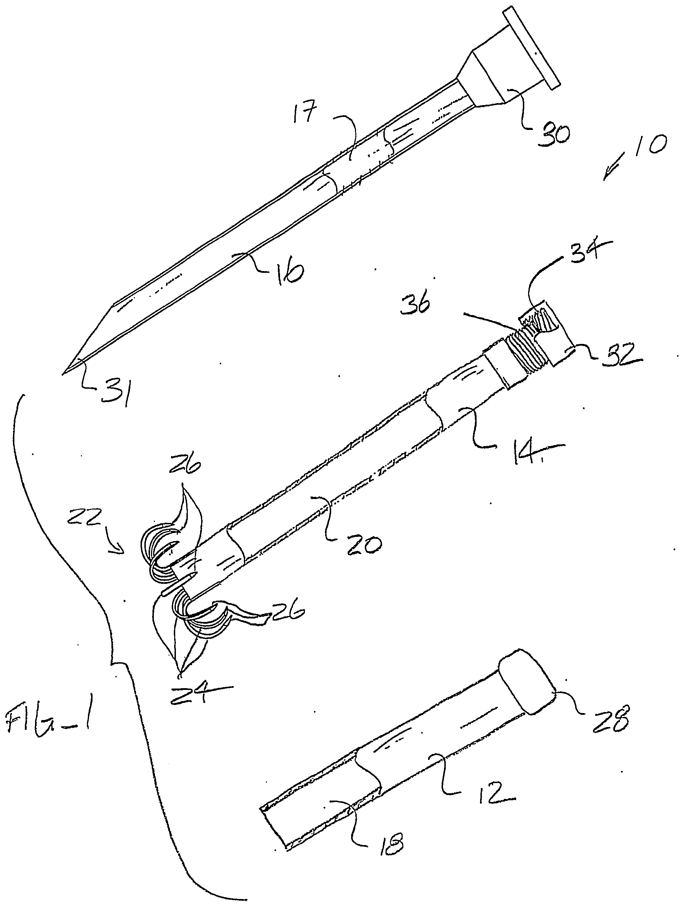

Referring to Fig. 1, a pericardial access system 10 comprises an outer tube 12, an inner tube 14, and an access needle 16. The outer tube 12 has an axial passage 18 extending therethrough. Similarly, the inner tube 14 has an axial passage 20 extending therethrough. The access needle 16 is hollow and will also include an inner lumen 17.

The inner tube 14 further includes a pericardial interface structure 22 formed on or otherwise coupled to a distal end of the tube. Pericardial interface structure 22 typically comprises a plurality of tissue-engaging tines 24, where the tines may have tissue-penetrating tips 26, at least in the first embodiment illustrated in Fig. 1. The tissue-engaging tines 24 are typically elastic or resilient, often being formed from an elastic or superelastic metal. In specific embodiments, individual tines 24 will be curved when unconstrained so that the tines may evert after release from constraint, as described hereinbelow.

The outer tube 12 typically comprises a hub 28 or other feature allowing manual manipulation in the outer tube. The access needle 16 will also include a hub 30 at its proximal end. The distal end or tip of the needle 16 will be sharpened or otherwise formed into a tissue-penetrating tip 31.

A proximal end of the inner tube 14 will preferably be formed with an adjustable needle stop 32. By adjustable, it is meant that the needle stop will be axially positionable relative to the remainder of the inner tube 20. By selecting an axial position of the needle stop 32, the advancement of the needle can be controlled as the needle stop will block further advancement of the hub 30 of the access needle 16. Usually, the axial adjustability of the needle stop 32 is achieved with a threaded connection, e.g. with internal threads 34 on the needle stop 32 (which is formed as a cylindrical cap) which engage external threads 36 on a proximal end of a main body of the inner tube 20.

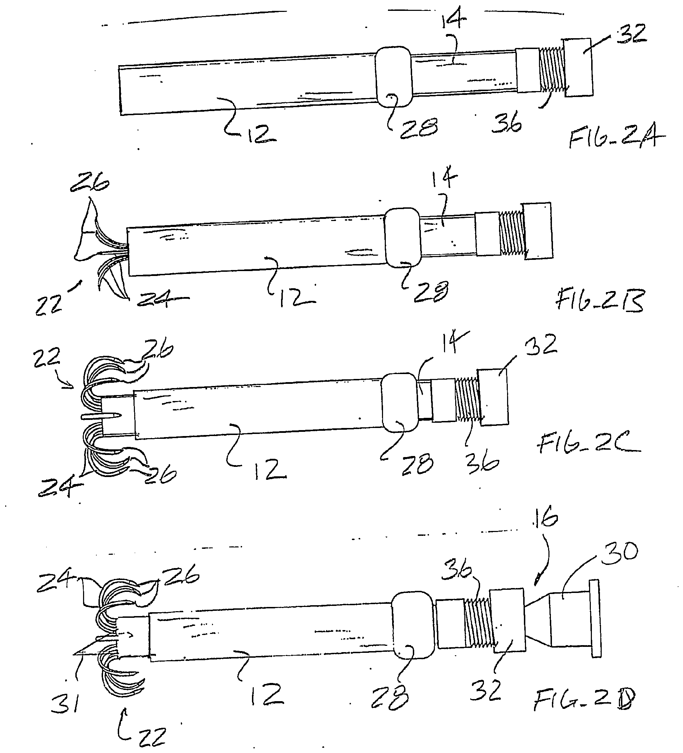

As shown in Figs. 2A through 2D, the inner tube 14 is typically received in the axial passage 18 (Fig. 1) of the outer tube 12. In an initial state or condition, the pericardial interface surface 22 will be restrained within a distal end of the outer tube 12, as shown in Fig. 2A.

By axially advancing the inner tube 14 relative to the outer tube 12, the tissue-engaging tines 24 of the pericardial interface structure 22 will emerge from the distal end of the outer tube. As the tines 24 emerge, they will evert in a radially outward direction, with sharpened tips 26 of the tines leading of the way. In this way, as described in more detail later, the sharpened tips may penetrate and capture the parietal pericardium or other tissue surface as the tines are advanced.

As the inner tube 14 is further axially advanced in a distal direction, as shown in Fig. 2C, the distal end of the inner tube 12 will fully emerge, allowing the tines 24 of the pericardial interface structure 22 to fully deploy and evert.

As shown in Fig. 2D, the needle 16 can be advanced through the inner tube 14 of the pericardial access system 10 so that the distal tip 31 emerges from the distal end of the inner tube beyond a distal-most region or surface of the pericardial interface structure 22. The extent (distance) to which the sharp tip 31 emerges from the inner tube 14 depends on the position of the needle stop 32 which can be adjusted by rotating the needle stop on the external threads 36 of the inner tube.

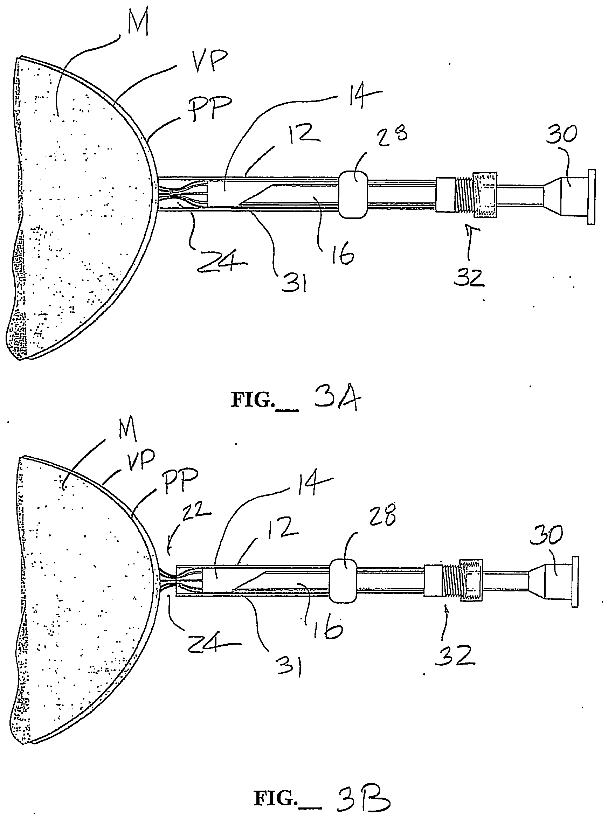

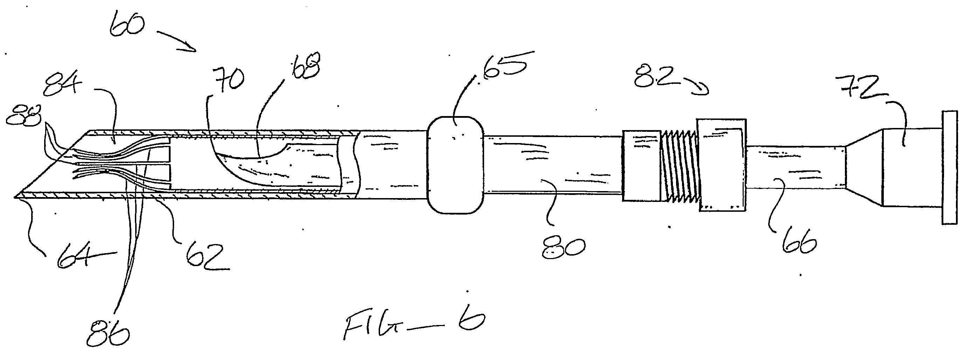

Referring now to Figs. 3A through 3E, use of the pericardial access system 10 for accessing an enlarged pericardial space will be described. As shown in Fig. 3A, a distal end of the outer tube 12 is initially engaged against a target location on the exterior of the parietal pericardium PP. For such access, it is desirable that the distal end of the outer tube be atraumatic, e.g. using a cannula having an atraumatic distal tip. In particular, it is desirable not to use an outer tube with a tissue-penetrating tip as shown, for example, in the embodiments of Fig. 6 described below.

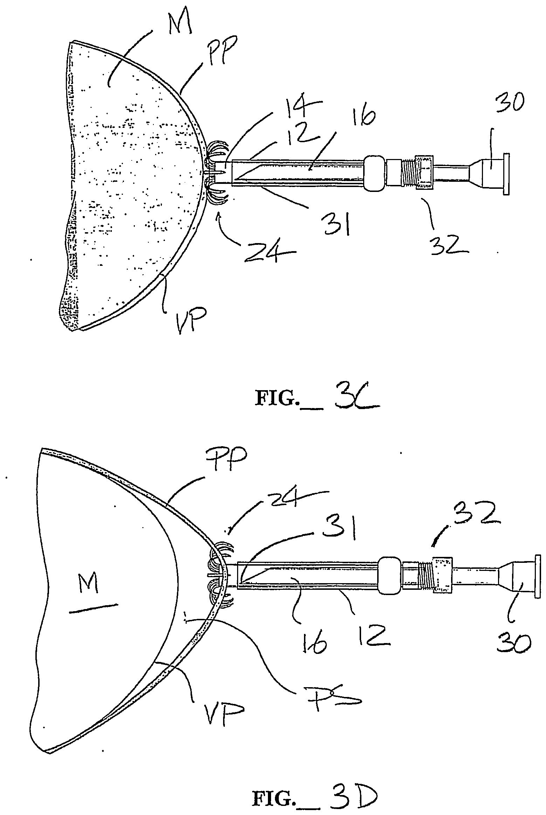

After the outer tube 12 initial contacts the parietal pericardium, the inner tube 14 is axially advanced so that the tissue engaging tines 24 of the pericardial interface structure 22 emerge and begin to evert, as show in in Fig. 3B. As the distal tips 26 of the tissue-engaging tines 24 are typically sharpened into tissue-penetrating points (Fig. 1), the tips will penetrate the parietal pericardium PP with minimal or no penetration into the underlying visceral pericardium VP, as shown in Fig. 3C.

Once the tissue-penetrating tines 24 have fully everted, as shown in Fig. 3C, the entire assembly of the pericardial access system 10 can be retracted so that the parietal pericardium PP can be drawn away from the visceral pericardium VP to create an enlarged pericardial space PS, as shown in Fig. 3D.

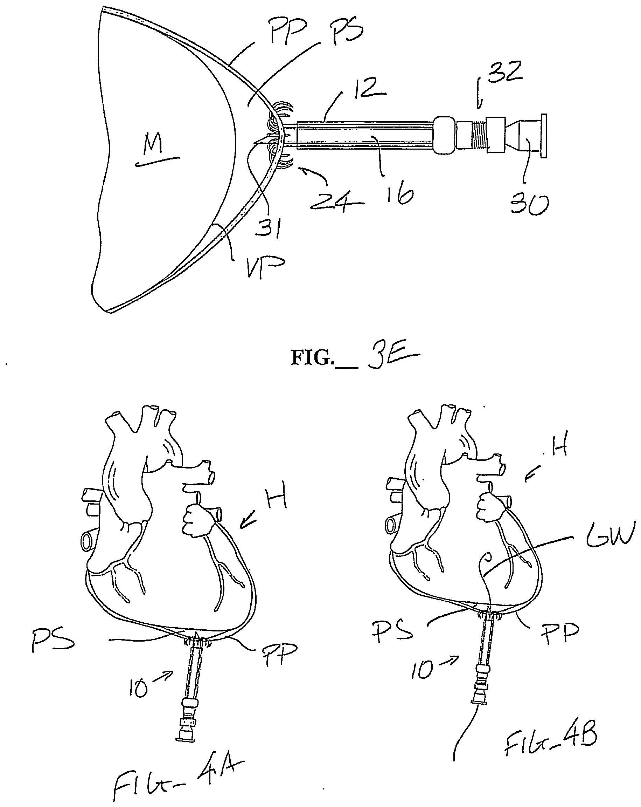

Once the enlarged pericardial space PS has been created, the sharpened tip 31 of the needle 16 can be advanced to penetrate the parietal pericardium PP, as shown in Fig. 3E. The lumen in the needle is then available for introducing tools, drugs, guidewires, or for any other purpose facilitated or enabled by needle access into an enlarged pericardial space.

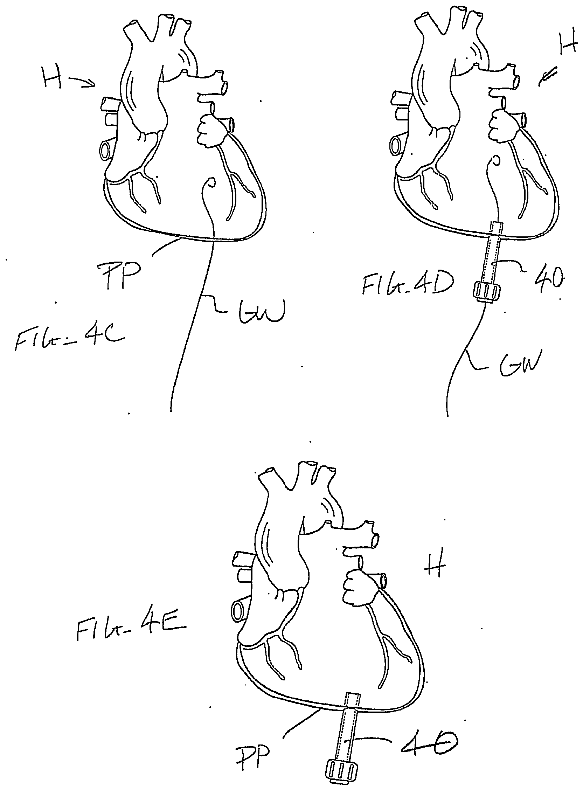

For example, as shown in Figs. 4A through 4E, a guidewire GW may be introduced into the pericardial space PS and advanced to a desired location. Usually, as shown in Fig. 4A, pericardial access system is used to capture and pull the parietal pericardium PP away from the visceral pericardium VP to provide the enlarged pericardial space PS, as described. A guidewire GW can then be introduced through the pericardial access system 10 and advanced into the pericardial space PS, as shown in Fig. 4B. Once the guidewire GW is sufficiently advanced into the pericardial space PS, the pericardial access system may be proximally withdrawn over the proximal section of the guidewire GW, as shown in Fig. 4C, leaving the guidewire GW in place for subsequent use. As shown in Fig. 4D, a conventional access tube 40 may then be introduced over the guidewire GW and advanced into the pericardial space PS to provide access for other tools or purposes, as shown in Figs. 4D and 4E.

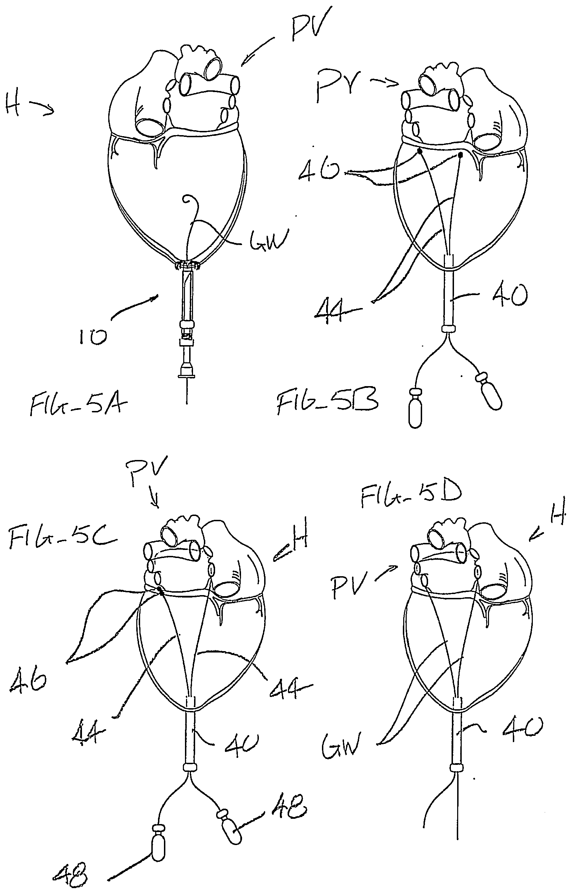

Referring now to Figs. 5A though 5D, the guidewire GW introduced into through the pericardial access system 10 (as shown in Fig. 5A), may be used to introduce an access sheath 40 which in turn may be used to introduce a pair of deflectable guidewires GW into the pericardial space PS and toward the pulmonary veins PV, as shown in Fig. 5B. Using control handles 48 for steering, one or both of the guidewires 44 may be looped around the pulmonary veins PV and the magnets 46 coupled together, as shown in Figs. 5C. The magnetic guidewires 44 may be then exchanged for a continuous guidewire GW, as shown in Fig. 5D, and the continuous guidewire used for a variety of purposes, such as for advancing an ablation catheter to perform pulmonary vein ablation.

Referring to Fig. 6, an alternative embodiment of a pericardial access system 60 will be described. The pericardial access system 60 comprises an outer tube 62. The outer tube 62 differs from outer tube 12 of pericardial access system 10 in that outer tube 62 has a tissue-penetrating tip 64 which allows self-introduction of the pericardial access system 60.

The pericardial access system 60 further includes a hub 65 at the proximal end of the outer tube 62 and an access needle 66 having a side port 68 adjacent to a tissue-penetrating distal tip 70. Inner tube 80 of pericardial access system 60 may be similar to inner tube 12 of pericardial access system 10. Typically, inner tube 80 will include a pericardial interface structure 84 at its distal end, where the interface structure includes a plurality of tissue-engaging tines 86 having distal tips 88. While in some instances the distal tips 86 might be sharpened or otherwise self-penetrating, the tissue interface structure 84 is intended primarily to be atraumatic, so the distal tips will often be blunt or otherwise made atraumatic to limit penetration into or other injury of the pericardium.

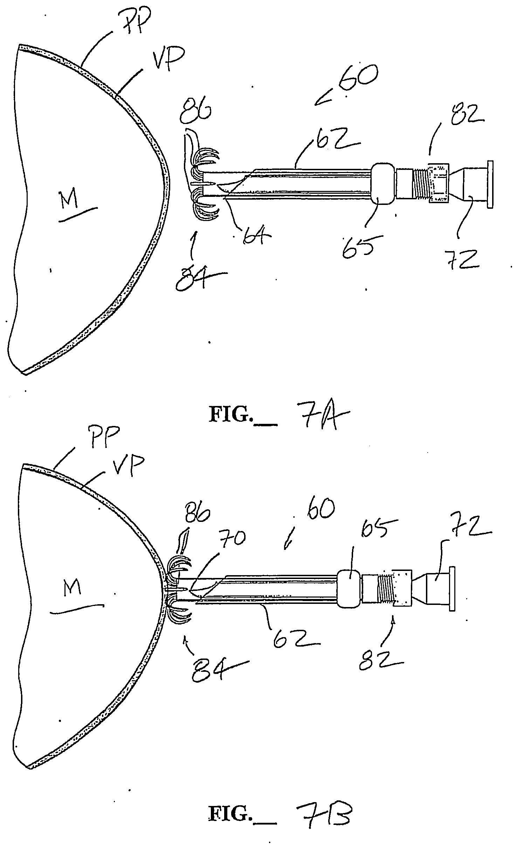

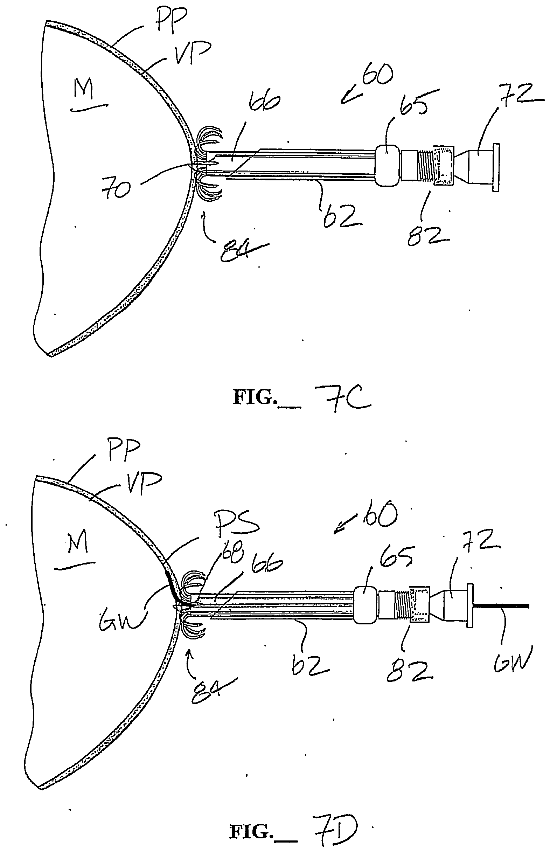

Pericardial access system 60 may be used to access a pericardial space as shown in Fig. 7A through 7D. Initially, the assembly of the pericardial access system 60, as shown for example in Fig. 6, may be self introduced percutaneously through a target site in the patient's thorax, for example, in a sub-xiphoid approach or an intercostal approach. Once the assembly of the pericardial access system has been introduced, the sharpened distal end 64 of the outer tube 60 will be retracted over the inner tube 80, as shown in Fig. 7A. Such retraction will simultaneously cause deployment of the tissue-engaging tine 86 of the pericardial interface structure 84. In contrast to previously described protocols, however, the deployment of the pericardial interface structure 84 will be done above the pericardium and the heart so that the distal tips 88 of the tines 86 do not penetrate the pericardium.

Once the pericardial interface structure 84 is deployed, a front or leading segment of the interface structure may be atraumatically engaged against the exterior of the pericardium, as shown in Fig. 7B. The access needle 66 may then be advanced so that the tissue-penetrating tip 70 penetrates the parietal pericardium PP, as shown in Fig. 7C. Usually, minimal or no penetration of the underlying visceral pericardium VP or myocardium M will be effected. The side port 68 on the access needle 66 allows a guidewire GW to be laterally deployed into the pericardial space PS even without enlargement of the space. The guidewire can then be advanced to a desired location and then the pericardial access system 60 then exchanged for a conventional access tube as described previously. Preferred embodiments of this invention, or embodiments useful for the understanding of the invention, are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, scope of the invention is solely defined by the appended claims. Moreover, any combination of the above-described elements in all possible variations thereof is encompassed by the present disclosure unless otherwise indicated herein or otherwise clearly contradicted by context.

## Claims

1. A pericardial access device comprising:
an outer tube (62);
an inner tube (80) axially slidably received in an axial passage of the outer tube (62);
a pericardial interface structure (84) disposed on a distal end of the inner tube (80) and having a plurality of tissue-engaging tines (86) configured to deploy from a constrained configuration to a deployed configuration as the distal end of the inner tube (80) is advanced to extend distally relative to the distal end of the outer tube (62), wherein the plurality of tissue-engaging tines (86) have atraumatic tips (88) which in the deployed configuration are configured to atraumatically engage tissue without tissue penetration; and
an access needle (66) having a tissue-penetrating tip (70) on a distal end thereof, said needle (66) being slidably received in an axial passage of the inner tube (80).

2. The pericardial access device of claim 1, wherein the leading or distal surfaces of the tissue-engaging tines (86) provide the atraumatic tip for engaging the parietal pericardium without tissue penetration.

3. The pericardial access device of any one of the preceding claims, wherein the plurality of tissue-engaging tines (86) are disposed about a periphery of a distal end of the inner tube (800 and wherein each tine (86) is straightened when constrained in the outer tube (62) and everts radially outwardly as it is advanced from the distal end of the outer tube (62).

4. The pericardial access device of any one of the preceding claims wherein the outer tube (62) comprises a cannula having a blunt end.

5. The pericardial access device of any one of claims 1 to 3 wherein the outer tube (62) comprises a needle having a tissue-penetrating end.

6. The pericardial access device of any one of the preceding claims further comprising an adjustable needle stop mechanism (32) to adjustably limit advancement of the access needle (66) from a distal end of the inner tube (80).

7. The pericardial access device of claim 6 wherein the needle stop mechanism (32) comprises a threaded end cap.

8. The pericardial access device of any one of the preceding claims wherein the access needle (66) has a side exit hole (68).

9. The pericardial access device of any one of the preceding claims further comprising a guidewire (44) configured to be advanced through the access needle (66) into a pericardial space.

## Patentansprüche

1. Perikard-Zugangs-Vorrichtung, das Folgende umfassend:
eine äußere Röhre (62);
eine innere Röhre (80), das axial verschiebbar in einem axialen Durchgang der äußeren Röhre (62) aufgenommen ist;
eine perikardiale Schnittstellen-Struktur (84), die an einem distalen Ende der inneren Röhre (80) angeordnet ist und eine Vielzahl von gewebe-eingreifenden Zinken (86) aufweist, die so konfiguriert sind, dass sie sich von einer eingeschränkten Konfiguration in eine entfaltete Konfiguration entfalten, wenn das distale Ende der inneren Röhre (80) vorwärts bewegt wird, um sich distal relativ zum distalen Ende der äußeren Röhre (62) zu erstrecken, wobei die Vielzahl von gewebe-eingreifenden Zinken (86) atraumatische Spitzen (88) aufweisen, die in der entfalteten Konfiguration so konfiguriert sind, dass sie atraumatisch in Gewebe eingreifen, ohne in Gewebe einzudringen; und
eine Zugangsnadel (66) mit einer gewebe-durchdringenden Spitze (70) an ihrem distalen Ende, wobei die Nadel (66) gleitend in einem axialen Durchgang der inneren Röhre (80) aufgenommen ist.

2. Perikard-Zugangs-Vorrichtung nach Anspruch 1, wobei die vorderen oder distalen Oberflächen der gewebe-eingreifenden Zinken (86) eine atraumatische Spitze zum Erfassen des parietalen Perikards ohne Gewebepenetration bereitstellen.

3. Perikard-Zugangs-Vorrichtung nach einem der vorhergehenden Ansprüche, wobei die Vielzahl von gewebe-eingreifenden Zinken (86) um einen Umfang eines distalen Endes der inneren Röhre (800) angeordnet sind und wobei jede Zinke (86) gerade ausgerichtet ist, wenn sie in die äußere Röhre (62) eingespannt ist, und sich radial nach außen verjüngt, wenn sie von dem distalen Ende der äußeren Röhre (62) vorgeschoben wird.

4. Perikard-Zugangs-Vorrichtung nach einem der vorhergehenden Ansprüche, wobei die äußere Röhre (62) eine Kanüle mit einem stumpfen Ende umfasst.

5. Perikard-Zugangs-Vorrichtung nach einem der Ansprüche 1 bis 3, wobei die äußere Röhre (62) eine Nadel mit einem gewebe-durchdringenden Ende umfasst.

6. Perikard-Zugangs-Vorrichtung nach einem der vorhergehenden Ansprüche, ferner umfassend einen einstellbaren Nadelanschlag-Mechanismus (32), um das Vorschieben der Zugangsnadel (66) von einem distalen Ende der inneren Röhre (80) aus einstellbar zu begrenzen.

7. Perikard-Zugangs-Vorrichtung nach Anspruch 6, wobei der Nadelanschlag-Mechanismus (32) eine Endkappe mit Gewinde umfasst.

8. Perikard-Zugangs-Vorrichtung nach einem der vorhergehenden Ansprüche, wobei die Zugangsnadel (66) ein seitliches Austrittsloch (68) aufweist.

9. Perikard-Zugangs-Vorrichtung nach einem der vorhergehenden Ansprüche, ferner umfassend einen Führungsdraht (44), der so konfiguriert ist, dass er durch die Zugangsnadel (66) in einen Perikard-Raum vorgeschoben werden kann.

## Revendications

1. Dispositif d'accès péricardique comprenant :
un tube externe (62) ;
un tube interne (80) reçu de manière coulissante et axiale dans un passage axial du tube externe (62) ;
une structure d'interface péricardique (84) disposée sur une extrémité distale du tube interne (80) et ayant une pluralité de fourchons de prise avec un tissu (86) configurés pour se déployer d'une configuration contrainte à une configuration déployée quand l'extrémité distale du tube interne (80) est avancée pour s'étendre de manière distale par rapport à l'extrémité distale du tube externe (62), dans lequel la pluralité de fourchons de prise avec un tissu (86) ont des pointes atraumatiques (88) qui dans la configuration déployée sont configurées pour entrer en prise de manière atraumatique avec un tissu sans pénétrer dans le tissu ; et
une aiguille d'accès (66) ayant une pointe de pénétration de tissu (70) sur une extrémité distale de celle-ci, ladite aiguille (66) étant reçue de manière coulissante dans un passage axiale du tube interne (80).

2. Dispositif d'accès péricardique selon la revendication 1, dans lequel les surfaces d'attaque ou distales des fourchons de prise avec un tissu (86) fournissent la pointe atraumatique destinée à entrer en prise avec le péricarde pariétal sans pénétrer dans le tissu.

3. Dispositif d'accès péricardique selon l'une quelconque des revendications précédentes, dans lequel la pluralité de fourchons de prise avec un tissu (86) sont disposés autour d'une périphérie d'une extrémité distale du tube interne (800) et dans lequel chaque fourchon (86) est redressé quand il est contraint dans le tube externe (62) et s'évase vers l'extérieur de manière radiale quand il est avancé depuis l'extrémité distale du tube externe (62).

4. Dispositif d'accès péricardique selon l'une quelconque des revendications précédentes dans lequel le tube externe (62) comprend une canule ayant une extrémité émoussée.

5. Dispositif d'accès péricardique selon l'une quelconque des revendications 1 à 3 dans lequel le tube externe (62) comprend une aiguille ayant une extrémité de pénétration de tissu.

6. Dispositif d'accès péricardique selon l'une quelconque des revendications précédentes comprenant en outre un mécanisme d'arrêt d'aiguille ajustable (32) pour limiter de manière ajustable l'avancée de l'aiguille d'accès (66) depuis une extrémité distale du tube interne (80) .

7. Dispositif d'accès péricardique selon la revendication 6 dans lequel le mécanisme d'arrêt d'aiguille (32) comprend un capuchon d'extrémité fileté.

8. Dispositif d'accès péricardique selon l'une quelconque des revendications précédentes dans lequel l'aiguille d'accès (66) a un orifice de sortie latéral (68) .

9. Dispositif d'accès péricardique selon l'une quelconque des revendications précédentes comprenant en outre un fil-guide (44) configuré pour être avancé à travers l'aiguille d'accès (66) dans un espace péricardique.
